# EUROPEAN PATENT APPLICATION

(11) **EP 4 767 836 A1**
(43) Date of publication of application: **01.07.2026**
(21) Application number: 24856540.0
(22) Date of filing: 23.08.2024
(51) Int. Cl.: A23L 33/10, A61K 8/9706, A61K 36/02, A61P 17/14, A61P 43/00, A61Q 7/00, C12N 1/12

(54) **AGENT FOR ACTIVATING HAIR DERMAL PAPILLA CELLS**

(30) Priority: 24.08.2023 JP 2023136488
(71) Applicant: Rohto Pharmaceutical Co., Ltd., Osaka-shi, Osaka 544-8666 (JP)
(72) Inventor: YAMADA, Kotaro, Osaka-shi, Osaka 544-8666 (JP); SOEJIMA, Yoshiomi, Osaka-shi, Osaka 544-8666 (JP); NAKAHARA, Ken, Osaka-shi, Osaka 544-8666 (JP)
(74) Representative: Schön, Christoph
(86) International application number: PCT/JP2024/029989
(87) International publication number: WO 2025/041852

(57) **Abstract**

An object of the present invention is to provide a formulation effective for activation of hair dermal papilla cells. Provided are a composition for activation of hair dermal papilla cells, a composition for hair regrowth promotion or hair growth, a composition for hair quality improvement, and a composition for FGF-2, FGF-7 or VEGF production promotion, containing a culture supernatant and/or extracellular vesicles of a microalga.

## Description

### TECHNICAL FIELD

The present invention relates to an agent for activating hair dermal papilla cells.

### BACKGROUND ART

The hair cycle (hair growth cycle) includes the growth phase, the regression phase, and the resting phase. The hair is repeatedly grown and fallen. Various types of cells are involved in the hair cycle. Among them, hair dermal papilla cells play a role of a leader. Hair dermal papilla cells are cells present in the central bottom of hair tissue, and play an important role in hair differentiation, such as proliferation promotion of surrounding hair matrix cells.

When the function of hair dermal papilla cells decreases due to various causes such as aging, stress in life, change in hormone balance, and side effects of drugs, activation and differentiation of hair matrix cells are suppressed. As a result, the hair cycle may be shortened so that hair loss may occur.

In recent years, regardless of men and women, the number of people suffering from thin hair or hair fall tends to increase due to the above-described various factors. In response to such a social situation, various hair regrowth promoters and hair growth agents have been developed.

In the pharmaceutical field, for example, a formulation containing minoxidil (6-(1-piperidinyl)-2,4-pyrimidinediamine-3-oxide) is put on the market as a therapeutic agent for AGA (male pattern hair loss).

Minoxidil is known to promote blood circulation by dilating peripheral blood vessels and promote hair growth by stimulating hair dermal papilla cells.

Effects of various components other than pharmaceutical components on hair regrowth or hair growth have been studied. For example, Patent Document 1 discloses that taurine prolongs the hair growth phase, and thus is excellent in hair growth promoting effect.

Patent Document 2 discloses that since a rice extract promotes production of a hepatocyte growth factor, the rice extract is excellent in hair growth promoting effect.

Patent Document 3 discloses that since a bamboo shoot extract activates hair dermal papilla cells and promotes production of a vascular endothelial cell growth factor and a fibroblast growth factor, the bamboo shoot extract is excellent in hair growth promoting effect.

### PRIOR ART DOCUMENT

### PATENT DOCUMENT

Patent Document 1: JP-A-2002-097116
Patent Document 2: JP-A-2004-099503
Patent Document 3: JP-A-2013-180999

### SUMMARY OF THE INVENTION

### PROBLEMS TO BE SOLVED BY THE INVENTION

As described above, it is presumed that a component capable of activating hair dermal papilla cells is effective for promoting hair regrowth and hair growth, but it has not yet been sufficiently reported which component is suitable for addition as an agent for activating hair dermal papilla cells.

Accordingly, an object of the present invention is to provide a formulation effective for activation of hair dermal papilla cells.

### MEANS FOR SOLVING THE PROBLEMS

In order to solve the above problems, the present inventors have extensively conducted studies, and resultantly found that a culture supernatant and/or extracellular vesicles of a microalga have an action on activation of hair dermal papilla cells, thereby completing the present invention.

That is, the present invention provides the following composition.
[1] A composition for activation of hair dermal papilla cells, containing a culture supernatant and/or extracellular vesicles of a microalga.
[2] A composition for hair regrowth promotion or hair growth, containing a culture supernatant and/or extracellular vesicles of a microalga.
[3] A composition for hair quality improvement, containing a culture supernatant and/or extracellular vesicles of a microalga.
[4] A composition for FGF-2, FGF-7 or VEGF production promotion, containing a culture supernatant and/or extracellular vesicles of a microalga.
[5] The composition according to any of [1] to [4], in which the microalga is a microalga belonging to the genus Pavlova, Euglena, Spirulina, or Chlorella.
[6] The composition according to any of [1] to [5], which is a food or beverage product, a cosmetic product, a pharmaceutical product, or a quasi-pharmaceutical product.
[7] Use of a culture supernatant and/or extracellular vesicles of a microalga in production of a composition for use in activation of hair dermal papilla cells.
[8] Use of a culture supernatant and/or extracellular vesicles of a microalga in production of a composition for use in hair regrowth promotion or hair growth.
[9] Use of a culture supernatant and/or extracellular vesicles of a microalga in manufacture of a composition for use in hair quality improvement.
[10] Use of a culture supernatant and/or extracellular vesicles of a microalga in manufacture of a composition for use in production promotion of FGF-2, FGF-7 or VEGF.
[11] Use according to any of [7] to [10], in which the microalga is a microalga belonging to the genus Pavlova, Euglena, Spirulina, or Chlorella.
[12] The use according to any of [7] to [11], wherein the composition is a food or beverage product, a cosmetic product, a pharmaceutical product, or a quasi-pharmaceutical product.

### EFFECT OF THE INVENTION

According to the present invention, hair regrowth and hair growth can be promoted by using a culture supernatant and/or extracellular vesicles of a microalga.

### BRIEF DESCRIPTION OF THE DRAWINGS

Fig. 1 is a graph showing the results of a test of addition of extracellular vesicles derived from Pavlova sp. to hair dermal papilla cells in Test Example 1.
Fig. 2 is a graph showing the results of a test of addition of extracellular vesicles derived from Arthrospira platensis to hair dermal papilla cells in Test Example 1.
Fig. 3 is a graph showing the results of a test of addition of extracellular vesicles derived from Chlorella vulgaris to hair dermal papilla cells in Test Example 1.

### MODE FOR CARRYING OUT THE INVENTION

### [Composition for activation of hair dermal papilla cells]

In one embodiment, the composition for activation of hair dermal papilla cells of the present invention contains a culture supernatant and/or extracellular vesicles of a microalga.

### (Culture supernatant and/or extracellular vesicles of microalga)

In the present description, "microalgae" refer to organisms having a cell size of 0.1 µm to 1000 µm in diameter among the rest of organisms performing photosynthesis other than mosses, ferns, and seed plants that generate oxygen. Also included are labyrinthulas, which are protists closely related to microalgae.

Examples of the microalgae include organisms of prokaryotes such as cyanobacteria, and eukaryotes such as Glaucophyta, Rhodophyta (red algae), Chlorophyta, Cryptophyta, Haptophyta, Heterokontophyta, Dinophyta, Euglenida, and Chlorarachniophyta.

Examples of the microalgae include microalgae belonging to the genus Pavlova, Euglena (Protozoa/green algae), Spirulina (blue-green algae), Chlorella (green algae), Dunaliella (green algae), Nannochloropsis (eustigmatophyceans), Scenedesmus (green algae), Botryococcus (green algae), and the Thraustochytrid of Labyrinthula. The microalgae exemplified above may be used alone or in combination of two or more thereof.

Among the microalgae, from the viewpoint of remarkably exhibiting the effect of the present invention, microalgae belonging to at least one species selected from the group consisting of the genus Pavlova, Euglena, Spirulina, and Chlorella are preferable, microalgae belonging to at least one species selected from the group consisting of the genus Pavlova, Euglena, and Spirulina are more preferable, microalgae belonging to at least one species selected from the group consisting of the genus Pavlova and Euglena are still more preferable, and microalgae belonging to the genus Pavlova are particularly preferable.

Pavlova is included in the phylum of the Haptophyta, and examples of the microalgae of the genus Pavlova include P. calceolate, P. granifera, P. gyrans, P. lutheri, P. pinguis, and P. salina. Among them, P. granifera and/or P. gyrans are preferable.

Examples of the microalgae of the genus Euglena include E. gracilis, E. longa, E. caudata, E. oxyuris, E. tripteris, E. proxima, E. viridis, E. sociabilis, E. ehrenbergii, E. deses, E. pisciformis, E. spirogyra, E. acus, E. geniculata, E. intermedia, E. mutabilis, E. sanguinea, E. stellata, E. terricola, E. klebsi, E. rubra, and E. cyclopicola. Among them, E. gracilis and/or E. longa are preferable.

The microalgae of the genus Spirulina may be, for example, the genus Arthrospira, a scientific name changed from the genus Spirulina, and examples thereof include S. platensis (A. platensis) and S. maxima (A. maxima). Among them, S. platensis is preferable.

Examples of the microalgae of the genus Chlorella include C. vulgaris, C. saccharophila, C. Ellipsoidea, C. pyrenoidosa, C. sorokiniana, and C. lobophora. Among them, C. vulgaris is preferable.

The microalgae exemplified above are widely distributed in sea water, fresh water such as in ponds and marshes, and brackish water. The microalgae may be separated from the water and then used. Alternatively, any microalgae already isolated may be used.

The microalgae exemplified above include related species and mutant strains thereof as long as the effect of the present invention is exhibited. Examples of the mutant strain include those obtained by a genetic method such as genetic recombination, transduction, or transformation.

In the present description, the culture supernatant of a microalga refers to a supernatant of a culture solution after the microalga is cultured. Examples of the supernatant include a supernatant of a culture solution in which a microalga has been cultured by a known culture method and from which the microalga has been removed by a known separation means such as centrifugation.

Culture conditions for preparing the culture supernatant of a microalga are not particularly limited as long as the effect of the present invention is exhibited, and a known method can be used. The following examples are given.

### (Culture conditions)

The microalga can be cultured using a culture solution. To the culture solution, a carbon source is added as a nutrient source, and as the carbon source, an inorganic carbon source (CO₂, NaHCO₃, Na₂CO₃, etc.), an organic carbon source (glucose or the like), or the like is used. The culture solution is not limited, but an autotrophic medium not containing an organic carbon source such as glucose as a nutrient source is preferably used. Examples of the culture solution include a culture solution to which a nutrient salt such as a nitrogen source, a phosphorus source, or a mineral is added, such as a Cramer-Myers medium or a modified Cramer-Myers medium.

When sea water is used depending on the type of microalgae, a culture medium for marine microalgae, artificial sea water, or the like can also be used, or a commercially available product such as an IMK culture medium can also be used.

The pH of the culture solution is not particularly limited as long as the effect of the present invention is exhibited, and examples thereof include 2 to 8, 3 to 8, 4 to 8, 5 to 8, 2 to 7.5, 3 to 7.5, 4 to 7.5, and 5 to 7.5.

The culture temperature is not particularly limited as long as the effect of the present invention is exhibited, and examples thereof include 15 to 40°C, 20 to 34°C, and 23 to 28°C.

The culture period is not particularly limited as long as the effect of the present invention is exhibited, and examples thereof include 4 to 30 days, 4 to 20 days, and 5 to 15 days.

Depending on the type of microalgae, the type of light source, the presence or absence of light and darkness, aerobic conditions, anaerobic conditions, and the like can be appropriately selected.

In one embodiment, in the case of culturing a marine microalga such as Pavlova, a known method can be used as long as the effect of the present invention is exhibited, and for example, culturing can be performed under conditions satisfying at least one of the following conditions:
Seawater concentration: 50% seawater
Medium concentration: IMK medium 2-fold concentration
Amount of culture solution: 800 mL
Light source: Side surface: Fluorescent lamp (100 to 150 µmol)
Light/dark cycle: 12 hours each for light and dark pH: about pH 7.4 at the start of each culture Aeration: done
Mechanical stirring: not done
Culture period: done for a period of about 10 days.
Culture temperature: 25°C to 28°C.

### (Collection of culture supernatant)

After the microalga is cultured under the culture condition exemplified above, the culture supernatant is collected using a known separation means such as centrifugation, but the collection method is not particularly limited as long as the culture supernatant of the microalga can be appropriately collected.

In the present invention, extracellular vesicles of a microalga can also be used. In the present description, the extracellular vesicle refers to a vesicle having an average diameter of usually 10 nm to 1 µm and secreted from a microalga. Although not limited, when extracellular vesicles of a microalga are used in the composition of the present invention, it is preferable that cells themselves of a microalga be not contained.

The method for separating extracellular vesicles is not particularly limited, and a known separation means can be used. As the method for separating extracellular vesicles, for example, a technique such as centrifugation can be used, but the method is not limited thereto.

In one embodiment, the method for producing extracellular vesicles includes, but is not limited to, a method including (1) a step of centrifuging a culture supernatant of a microalga to yield a supernatant; and (2) a step of further centrifuging the yielded product as necessary to yield a precipitate; by which microalga-derived extracellular vesicles that exhibit an effective activity can be produced.

In an exemplary embodiment, the centrifugation in said steps (1) and (2) may be carried out for 30 to 60 minutes at a centrifugal force of, for example, 1,000 to 20,000 × g, 1,500 to 20,000 × g, 1,500 to 15,000 × g, or 1,500 to 10,000 × g. At this time, the centrifugation may be carried out stepwise by changing a speed or time. For example, the centrifugation may be performed at a low speed of 1,500 to 2,000 × g to separate a culture supernatant, followed by centrifugation at a high speed of 10,000 to 20,000 × g to further remove cells or cell-associated remnants, residues, and the like.

### [Application]

In one embodiment, the composition of the present invention is suitably used for activation of hair dermal papilla cells. Hair dermal papilla cells are present in the central bottom of hair tissue, secrete various growth factors, angiogenic factors, and the like by the cells' own activation, and promote proliferation of surrounding hair matrix cells and supply of nutrients to hair matrix cells. A formulation containing minoxidil has been put on the market as a therapeutic agent for AGA (male pattern hair loss). The hair regrowth effect of minoxidil is considered to be caused, at least in part, by activation of hair dermal papilla cells. For example, VEGF (vascular endothelial growth factor) is known to decrease in expression in hair tissue of male pattern hair loss, but minoxidil can promote production of VEGF in hair dermal papilla cells. Therefore, it is presumed that a component capable of promoting VEGF production in hair dermal papilla cells promotes nutrient supply to surrounding hair matrix cells and contributes to hair regrowth and hair growth. In Examples (Test Examples) described later, the effect on VEGF-A has been confirmed. VEGF-A is known to develop capillary networks around hair follicles, enlarge hair follicle tissue, and be involved in hair growth promotion. In addition, since the composition of the present invention is suitably used for activation of hair dermal papilla cells and leads to improvement of the environment around hair follicles, it is expected to have an effect of improving not only male pattern hair loss but also female pattern hair loss (thinning hair of women) such as androgenic alopecia, alopecia areata, stress-derived alopecia, and alopecia in animals such as pets.

In addition, it is known that in hair dermal papilla cells of male pattern hair loss, the expression level of FGF-7 (fibroblast growth factor) gene decreases to about half. Adenosine for use in quasi-pharmaceutical products and the like is known to have an action of promoting production of FGF-7 and an action of promoting production of VEGF, and is considered to exhibit by these actions an effect on hair regrowth and hair growth. Therefore, it is presumed that a component capable of promoting FGF-7 production in hair dermal papilla cells promotes proliferation and differentiation of surrounding hair matrix cells and contributes to hair regrowth and hair growth.

In addition, FGF-2 is known to have a strong angiogenic action. The angiogenic action may exhibit an effect of VEGF or FGF-7 or more known in the action, and the involvement in hair regrowth and hair growth has attracted attention. Therefore, it is presumed that a component capable of promoting production of FGF-2 in hair dermal papilla cells, and furthermore a component capable of promoting production of FGF-2 and VEGF in hair dermal papilla cells together promote nutrient supply to surrounding hair matrix cells, and contribute to hair regrowth and hair growth.

It is known that IGF-1 (insulin-like growth factor), when produced from hair dermal papilla cells, can expand epithelial cells in hair follicles and contribute to prolongation of the growth phase in the hair cycle. This leads to long hair growth during the growth phase of the hair cycle and to growth and maintenance of the morphology of hair bulb. Therefore, it is presumed that a component capable of promoting production of IGF-1 in hair dermal papilla cells contributes to long and healthy growth of hair.

Activation of hair dermal papilla cells is effective not only in contributing to hair regrowth but also in quantitative and qualitative improvement of hair. As described above, when production of FGF-2 or VEGF is promoted, blood supply to hair tissue is improved by the angiogenic action, and hair is grown thick by growth of hair globule due to proliferation and differentiation of hair matrix cells, and hair is grown dense and glossy by acting on melanocytes adjacent to hair matrix cells. Therefore, it is presumed that a component capable of promoting production of at least one selected from the group consisting of FGF-2, FGF-7, IGF-1 and VEGF in hair dermal papilla cells contributes to improvement of hair quality.

The improvement of hair quality is not limited, and examples of the hair quality include at least one selected from the group consisting of thickness of hair, color density of hair, texture of hair, and hardness of hair.

In Examples (Test Examples) described later, it has been confirmed that a culture supernatant and/or extracellular vesicles of a microalga can promote production of at least one selected from the group consisting of FGF-2, FGF-7, IGF-1 and VEGF in hair dermal papilla cells. Therefore, a culture supernatant and/or extracellular vesicles of a microalga can be suitably used for activation of hair dermal papilla cells, suitably used for promotion of hair regrowth and hair growth by promoting production of the component, and further suitably used for improvement of hair quality.

The composition of the present invention can be added to or mixed with a pharmaceutical product, a quasi-pharmaceutical product, a cosmetic product, a food product, a beverage, a feed, or a pet food. Alternatively, the composition of the present invention can be used as it is as a pharmaceutical product, a quasi-pharmaceutical product, a cosmetic product, a food product, a beverage, a feed, a pet food, or the like.

When the composition of the present invention is added to or mixed with a so-called external preparation such as a pharmaceutical product, a quasi-pharmaceutical product, or a cosmetic product, the composition of the present invention can be used as a pharmaceutical product, a quasi-pharmaceutical product, or a cosmetic product on which activation of hair dermal papilla cells, hair regrowth, hair growth, promotion or improvement of hair quality, or the like is clearly indicated or implied as an efficacy, an effect or a function.

When the above functionality is not specified, for example, it is also possible to use the composition of the present invention by making indications to those for obtaining texture from the root, those for obtaining stiffness from the root, those concerned about the hairline, those for obtaining a sense of volume, those for obtaining hair roots that are difficult to fall off, those for obtaining early hair care, those for growing hair from the scalp, those for leading to a correct hair cycle, those for invigorating hair, those for adjusting the hair environment, those for stimulating scalp cells, those for supporting the roots of hair, those for obtaining a fluffy image, and those for improving the weak hair, and the like.

When used in a so-called external preparation such as a pharmaceutical product, a quasi-pharmaceutical product, or a cosmetic product, the composition of the present invention is preferably prepared as a hair regenerating product, a product for hair regrowth, a product for invigorating hair, a hair loss suppressing product, or the like.

When used in a so-called external preparation such as a pharmaceutical product, a quasi-pharmaceutical product, or a cosmetic product, the adult daily dose of extracellular vesicles of a microalga can be appropriately determined depending on the condition of the individual, body weight, sex, age, activity of the material, ingestion or route of administration, ingestion or administration schedule, formulation form, or other factors. Examples of the adult daily dose of extracellular vesicles of a microalga include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more. Examples of the adult daily dose of extracellular vesicles of a microalga include 5 mg or less, 3 mg or less, 1 mg or less, 900 µg or less, 800 µg or less, 700 µg or less, 600 µg or less, 500 µg or less, 400 ng or less, 300 µg or less, 200 µg or less, and 100 µg or less. In addition, examples of the adult daily dose of extracellular vesicles of a microalga include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg. In addition, the dose of extracellular vesicles of a microalga varies depending on various factors such as the degree of symptom progression, onset time, age, health condition, and complications of a subject, but when an adult is used as a reference, generally, 1 µg/kg to 200 mg/kg of the composition, or 50 µg/kg to 50 mg/kg in another aspect may be administered 1 to 3 times a day, and the dose does not limit the range of the present description by any method.

When used in a so-called external preparation such as a pharmaceutical product, a quasi-pharmaceutical product, or a cosmetic product, the adult daily dose of extracellular vesicles of a microalga can be appropriately determined depending on the condition of the individual, body weight, sex, age, activity of the material, ingestion or route of administration, ingestion or administration schedule, formulation form, or other factors. Examples of the adult daily dose of extracellular vesicles of a microalga include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more as the protein amount. Examples of the adult daily dose of extracellular vesicles of a microalga include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily dose of extracellular vesicles of a microalga include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg. In addition, the dose of extracellular vesicles of a microalga varies depending on various factors such as the degree of symptom progression, onset time, age, health condition, and complications of a subject, but when an adult is used as a reference, 1 µg/kg to 200 mg/kg of the composition, or 50 µg/kg to 50 mg/kg in another aspect may be administered 1 to 3 times a day, and the dose does not limit the range of the present description by any method.

In another embodiment, the adult daily dose of extracellular vesicles of a microalga is, for example, 1 × 10⁷ or more, 1 × 10⁸ or more, or 1 × 10⁹ or more in terms of the number of vesicles. In addition, the adult daily dose of extracellular vesicles of a microalga is, for example, 1 × 10¹⁴ or less, 1 × 10¹³ or less, 1 × 10¹² or less, 1 × 10¹¹ or less, or 1 × 10¹⁰ or less in terms of the number of vesicles. Examples of the adult daily dose of extracellular vesicles of a microalga include 1 × 10⁷ to 1 × 10¹⁴, 1 × 10⁷ to 1 × 10¹³, 1 × 10⁷ to 1 × 10¹², 1 × 10⁷ to 1 × 10¹¹, 1 × 10⁸ to 1 × 10¹⁴, 1 × 10⁸ to 1 × 10¹³, 1 × 10⁸ to 1 × 10¹², 1 × 10⁸ to 1 × 10¹¹, 1 × 10⁹ to 1 × 10¹⁴, 1 × 10⁹ to 1 × 10¹³, 1 × 10⁹ to 1 × 10¹², and 1 × 10⁹ to 1 × 10¹¹ in terms of the number of vesicles.

The adult daily dose of the culture supernatant of a microalga can be appropriately determined according to the condition of the individual, body weight, sex, age, activity of the material, administration schedule, formulation form, or other factors. The adult daily dose of the culture supernatant of a microalga can be, for example, an amount in which extracellular vesicles of a microalga are contained in the above-described intake amount or dose.

The composition of the present invention may be taken or administered in 1 to several portions per day, usually 1 to 6 times per day, 1 to 3 times per day, 1 to 2 times per day, or at any period and interval, but preferably once per day.

When the composition of the present invention is added to or mixed with a food product, a beverage, a feed, or a pet food, the composition of the present invention can be used as a food or beverage product on which activation of hair dermal papilla cells, hair regrowth, hair growth, promotion or improvement of hair quality, or the like is clearly indicated or implied as functionality, that is, as a health food product, a food product labeled with functionality, a food product for patients, and a food product for specified health uses. In addition, the composition of the present invention can be used as a so-called doctor's supplement recommended or presented by a doctor in a hospital and/or a doctor's office, or internal medicine, orthopedic surgery, animal hospital, or the like even when the functionality is not clearly indicated or implied.

When the above functionality is not specified, for example, it is also possible to use the composition of the present invention by making indications to those for obtaining texture from the root, those for obtaining stiffness from the root, those concerned about the hairline, those for obtaining a sense of volume, those for obtaining hair roots that are difficult to fall off, those for obtaining early hair care, those for growing hair from the scalp, those for leading to a correct hair cycle, those for invigorating hair, those for adjusting the hair environment, those for stimulating scalp cells, those for supporting the roots of hair, those for obtaining a fluffy image, and those for improving the weak hair, and the like.

The health food product, food product labeled with functionality, food product for patients, and food product for specified health uses can be produced using conventional means in the same manner as known pharmaceutical preparations.

The composition of the present invention can be applied in the form of oral use, internal use, and the like. When used as a pharmaceutical composition, the composition of the present invention may be used therapeutically or non-therapeutically.

The adult daily oral intake amount or dose of extracellular vesicles of a microalga can be appropriately determined depending on the condition of the individual, body weight, sex, age, activity of the material, ingestion or route of administration, ingestion or administration schedule, formulation form, or other factors. Examples of the adult daily oral intake amount or dose of extracellular vesicles of a microalga include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more. Examples of the adult daily oral intake amount or dose of extracellular vesicles of a microalga include 5 mg or less, 3 mg or less, 1 mg or less, 900 µg or less, 800 µg or less, 700 µg or less, 600 µg or less, 500 µg or less, 400 ng or less, 300 µg or less, 200 µg or less, and 100 µg or less. In addition, examples of the adult daily oral intake amount or dose of extracellular vesicles of a microalga include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg.

In another embodiment, the adult daily oral intake amount or dose of extracellular vesicles of a microalga is, for example, 1 × 10⁷ or more, 1 × 10⁸ or more, or 1 × 10⁹ or more in terms of the number of vesicles. In addition, the adult daily oral intake amount or dose of extracellular vesicles of a microalga is, for example, 1 × 10¹⁴ or less, 1 × 10¹³ or less, 1 × 10¹² or less, 1 × 10¹¹ or less, or 1 × 10¹⁰ or less in terms of the number of vesicles. Examples of the adult daily oral intake amount or dose of extracellular vesicles of a microalga include 1 × 10⁷ to 1 × 10¹⁴, 1 × 10⁷ to 1 × 10¹³, 1 × 10⁷ to 1 × 10¹², 1 × 10⁷ to 1 × 10¹¹, 1 × 10⁸ to 1 × 10¹⁴, 1 × 10⁸ to 1 × 10¹³, 1 × 10⁸ to 1 × 10¹², 1 × 10⁸ to 1 × 10¹¹, 1 × 10⁹ to 1 × 10¹⁴, 1 × 10⁹ to 1 × 10¹³, 1 × 10⁹ to 1 × 10¹², and 1 × 10⁹ to 1 × 10¹¹ in terms of the number of vesicles.

The adult daily oral intake amount or dose of the culture supernatant of a microalga can be appropriately determined depending on the condition of the individual, body weight, sex, age, activity of the material, ingestion or route of administration, ingestion or administration schedule, formulation form, or other factors. The adult daily oral intake amount or dose of the culture supernatant of a microalga can be, for example, an amount in which extracellular vesicles of a microalga are contained in the above-described intake amount or dose.

The adult daily oral intake amount or dose may be divided depending on the dosage form, for example, into 1 to 6 capsules, 1 to 4 capsules, 1 to 3 capsules, or 1 to 2 capsules, in the case of capsules.

The composition of the present invention may be taken or administered in 1 to several portions per day, usually 1 to 6 times per day, 1 to 3 times per day, 1 to 2 times per day, or at any period and interval, but preferably once per day.

When the present invention is used as a feed, a pet food, or the like, the target organism is not particularly limited, but is preferably mammal, reptile, amphibian, bird, or fish, more preferably mammal other than human. For example, the target organism includes platypus, spiny anteater, opossum, native cat, kangaroo, aardvark, hyrax, elephant, armadillo, sloth, anteater, tree shrew, flying lemur, chimpanzee, rabbit, degu, dormouse, squirrel, raccoon, mouse, hedgehog, chinchilla, ferret, camel, wild boar, giraffe, deer, cattle, goat, hippopotamus, whale, dolphin, horse, rhinoceros, tapir, bat, monkey, tiger, wolf, weasel, bear, seal, dog, cat, parakeet, parrot, finch, owl, and eared owl. Among them, dog or cat is more preferable.

When the present invention is used as a feed, a pet food, or the like, it may be fed several times a day by adding it to a staple food or the like, or may be given as a snack as needed.

### EXAMPLES

Next, a specific description is made of the present invention with reference to Examples, but the present invention is not limited to the following Examples.

### [Test example 1: Test of addition of culture supernatant and/or extracellular vesicles of a microalga to hair dermal papilla cells]

In a 24 well plate, hair dermal papilla cells were seeded at 6×10³ cells/well, and washed twice with 5 mL/dish of HBSS (hereinafter, simply referred to as a medium). Accutase (manufactured by Nacalai Tesque) was added at 1 mL/dish. The cells were cultured in a 5%CO₂ incubator at 37°C, and the medium was added at 3 mL/dish. The mixture was transferred to a 15 mL tube, followed by centrifugation at 220 × g for 5 minutes at 4°C. The supernatant was removed, and the pellet was suspended in the medium to measure the number of cells. The number of cells was adjusted for seeding at 1 mL/well. The cells were cultured in a 5%CO₂ incubator at 37°C.

Using a commercially available medium for algae, Pavlova sp., Arthrospira platensis, or Chlorella vulgaris was cultured by a conventional method, and ultracentrifugation treatment was performed according to the following procedure.

Each culture supernatant was transferred to an ultracentrifugation clear tube at 11 mL/tube. Ultracentrifugation treatment was performed using Optima XE-90 Ultracentrifuge (manufactured by BECKMAN COULTER) under conditions of ultracentrifugation, 35,000 rpm, 70 minutes, and 4°C. The supernatant was removed and the precipitate was suspended in PBS (-). Again, ultracentrifugation treatment was performed in the same manner under the conditions of 35,000 rpm, 70 minutes, and 4°C, and the supernatant was removed and the precipitate was suspended in PBS (-). The resulting samples were stored at 4°C.

The following samples were obtained as described above.
- Pavlova sp. culture supernatant-ultracentrifuged sample (protein concentration 2500 µg/mL)
- Arthrospira platensis culture supernatant-ultracentrifuged sample (protein concentration 226 µg/mL)
- Chlorella vulgaris culture supernatant-ultracentrifuged sample (protein concentration 84.4 µg/mL)

The sample or PBS (-) was mixed with the medium. Addition conditions of the sample are as follows.
- Pavlova sp. 40 µg/mL
- Arthrospira platensis 40 µg/mL
- Chlorella vulgaris 10 µg/mL

Human hair dermal papilla cells (HFDPC cells, manufactured by Cell Applications) seeded the day before were washed once. The mixed sample-containing medium was added at 250 µL/well. The cells were cultured in a 5%CO₂ incubator at 37°C for 48 hours.

The human hair dermal papilla cells after 48 hours of treatment of each sample, from which the medium was removed, were washed. QIAzol (manufactured by QIAGEN) was added at 700 mL/well. The mixture was collected in a 1.5 mL tube with a cell scraper and stored at -80°C. Thereafter, RNA was extracted with miRNeasy mini Kit (manufactured by QIAGEN).

The RNA sample was subjected to DNase treatment after measurement of the RNA concentration with NanoDrop ND-1000 (manufactured by Thermo Fisher Scientific), and then cDNAized through reverse transcription reaction.

The cDNA sample after completion of the reverse transcription was diluted 5 times with RNase free water and then stored at -30°C.

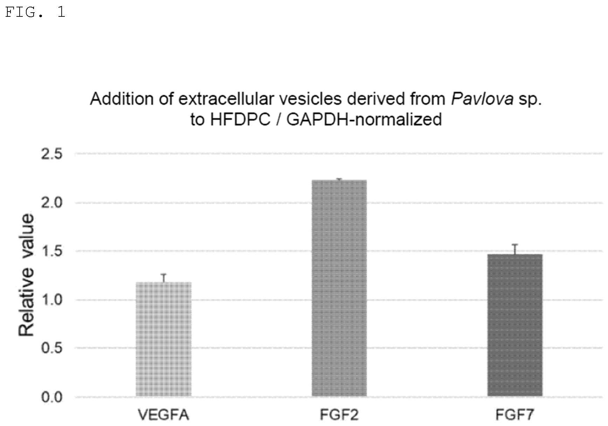

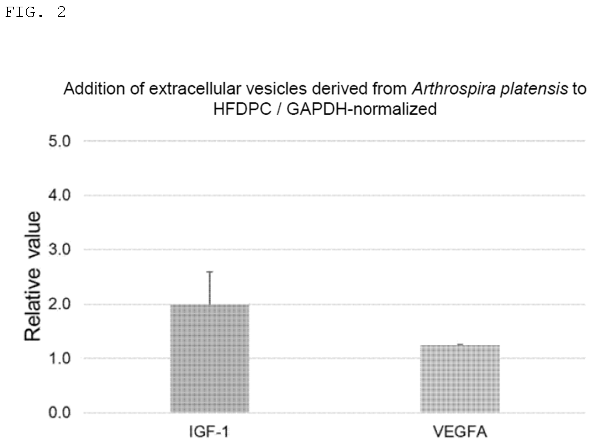

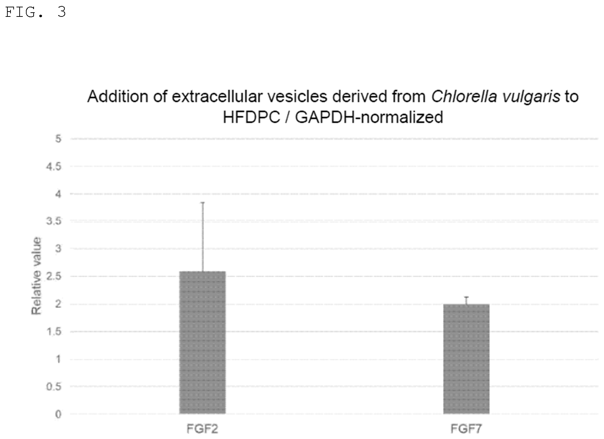

Gene expression analysis of FGF-2, FGF-7, IGF-1 and VEGF was performed by a real-time PCR method using a TaqMan probe by QuantStudio (registered trademark) 3 real-time PCR system (manufactured by Thermo Fisher Scientific). A case where each sample was not added was used as a control (a sample to which only PBS was added), and GAPDH was used as an internal standard. Data analysis was performed by using a ΔΔCT method. The results of the test of addition of the extracellular vesicles derived from Pavlova sp. to hair dermal papilla cells are shown in Fig. 1, the results of the test of addition of the extracellular vesicles derived from Arthrospira platensis to hair dermal papilla cells are shown in Fig. 2, and the results of the test of addition of the extracellular vesicles derived from Chlorella vulgaris to hair dermal papilla cells are shown in Fig. 3. Figs. 1 to 3 show the relative results when each sample was added with the control (sample to which only PBS was added) as 1.

As shown in Figs. 1 to 3, it has been confirmed that addition of the culture supernatant and/or extracellular vesicles of a microalga to hair dermal papilla cells can promote production of at least one selected from the group consisting of FGF-2, FGF-7, IGF-1 and VEGF in hair dermal papilla cells, and activates hair dermal papilla cells. Therefore, a culture supernatant and/or extracellular vesicles of a microalga can be suitably used for activation of hair dermal papilla cells, suitably used for promotion of hair regrowth and hair growth by promoting production of the component, and further suitably used for improvement of hair quality.

## Claims

1. A composition for activation of hair dermal papilla cells, comprising a culture supernatant and/or extracellular vesicles of a microalga.

2. A composition for hair regrowth promotion or hair growth, comprising a culture supernatant and/or extracellular vesicles of a microalga.

3. A composition for hair quality improvement, comprising a culture supernatant and/or extracellular vesicles of a microalga.

4. A composition for FGF-2, FGF-7 or VEGF production promotion, comprising a culture supernatant and/or extracellular vesicles of a microalga.

5. The composition according to any one of claims 1 to 4, wherein the microalga is a microalga belonging to the genus Pavlova, Euglena, Spirulina, or Chlorella.

6. The composition according to any one of claims 1 to 4, which is a food or beverage product, a cosmetic product, a pharmaceutical product, or a quasi-pharmaceutical product.
